(19) Europäisches Patentamt
European Patent Office
Office européen des brevets

(11) **EP 2 765 176 A2**

(12) **EUROPEAN PATENT APPLICATION**

(43) Date of publication:
13.08.2014 Bulletin 2014/33

(51) Int Cl.:
*C10G 1/00* (2006.01)       *C02F 1/28* (2006.01)
*C02F 1/42* (2006.01)       *C10L 1/02* (2006.01)
*B01J 49/00* (2006.01)      *B01J 47/00* (2006.01)
*B01J 47/02* (2006.01)      *C10B 53/02* (2006.01)

(21) Application number: 14151927.2

(22) Date of filing: 21.01.2014

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: 07.02.2013 US 201313762104

(71) Applicant: **KiOR, Inc.
Pasadena, TX 77507 (US)**

(72) Inventors:
• **RAMIREZ-CORREDORES, Maria Magdalena
Houston, TX 77062 (US)**

• **TONG, Xiaowei
Houston, TX 77054 (US)**
• **BANDA, Rocio Maria
Houston, TX 77089 (US)**
• **ROEMISCH, Royce
Houston, TX 77008 (US)**

(74) Representative: **Lucas, Brian Ronald
Lucas & Co.
135 Westhall Road
Warlingham,
Surrey CR6 9HJ (GB)**

(54) **Organics recovery from the aqueous phase of biomass catalytic pyrolysis**

(57)      A method for recovering a water-soluble complex mixture of organic compounds from an aqueous stream comprising:

a) passing said aqueous stream comprising a water-soluble complex mixture of organic compounds to a removal zone A for contact with a sorbent A comprising a polymeric microreticular sorbent resin for removal of at least a portion of the water-soluble complex mixture of organic compounds from said aqueous stream forming a removed quantity A comprising water-soluble organic compounds;

b) passing said aqueous stream from said removal zone A to a removal zone B for contact with a sorbent B for removal of at least a portion of the water-soluble complex mixture of organic compounds from said aqueous stream forming a removed quantity B comprising water-soluble organic compounds; and

c) recovering at least a portion of said removed quantity A from said removal zone A forming recovered quantity A and recovering at least a portion of said removed quantity B from said removal zone B forming recovered quantity B.

FIG. 1

**Description**

**FIELD OF THE INVENTION**

Embodiments of the invention relate generally to an improved method for recovering organics from the aqueous phase. More particularly, embodiments of the invention relate to a method including one or more removal zones/stages employing sorbents or extractants for removal of organics from such aqueous phase and to methods of recovering such organics after removal.

**INCORPORATION BY REFERENCE**

The entirety of the following patent application is hereby expressly incorporated herein by reference: US Patent Application No. 13/212,861 filed on August 18, 2011.

**BACKGROUND OF THE INVENTION**

With the rising costs and environmental concerns associated with fossil fuels, renewable energy sources have become increasingly important. The development of renewable fuel sources provides a means for reducing the dependence on fossil fuels. Accordingly, many different areas of renewable fuel research are currently being explored and developed.

With its low cost and wide availability, biomass has increasingly been emphasized as an ideal feedstock in renewable fuel research. Consequently, many different conversion processes have been developed that use biomass as a feedstock to produce useful biofuels and/or specialty chemicals. Existing biomass conversion processes include, for example, combustion, gasification, slow pyrolysis, fast pyrolysis, thermocatalytic pyrolysis, liquefaction, and enzymatic conversion. One of the useful products that may be derived from the aforementioned biomass conversion processes is a liquid product commonly referred to as "bio-oil." Bio-oil may be processed into transportation fuels, hydrocarbon chemicals, and/or specialty chemicals.

In the conversion of biomass to bio-oils, even after the separation of the reaction products into bio-oil and aqueous phases, significant amounts of water-soluble organic compounds can be present in the aqueous phase. The loss of these organic compounds to the aqueous phase results in a decrease in the overall yield of bio-oil.

Accordingly, there is a need for an improved method for recovering organics from an aqueous stream produced in the conversion of biomass to a bio-oil.

**BRIEF SUMMARY OF THE INVENTION**

In accordance with an embodiment of the present invention, a method for recovering a water-soluble complex mixture of organic compounds from an aqueous stream is provided including:

a) passing the aqueous stream comprising a water-soluble complex mixture of organic compounds to a removal zone A for contact with a sorbent A comprising a polymeric microreticular sorbent resin for removal of at least a portion of the water-soluble complex mixture of organic compounds from the aqueous stream forming a removed quantity A comprising water-soluble organic compounds;

b) passing the aqueous stream from the removal zone A to a removal zone B for contact with a sorbent B for removal of at least a portion of the water-soluble complex mixture of organic compounds from the aqueous stream forming a removed quantity B comprising water-soluble organic compounds; and

c) recovering at least a portion of the removed quantity A from the removal zone A forming recovered quantity A and recovering at least a portion of the removed quantity B from the removal zone B forming recovered quantity B.

In accordance with another embodiment of the present invention, a method for recovering a water-soluble complex mixture of organic compounds from an aqueous stream is provided and includes:

a) separating a bio-oil/water stream comprising a water-soluble complex mixture of organic compounds, water-insoluble organic compounds, and water into a bio-oil stream comprising water-insoluble organic compounds and into the aqueous stream comprising a water-soluble complex mixture of organic compounds;

b) contacting the aqueous stream with an activated carbon-based sorbent for removal of at least a portion of the water-soluble complex mixture of organic compounds from the aqueous stream forming a removed quantity comprising water-soluble organic compounds, wherein the activated carbon-based sorbent has been surface treated in a manner resulting in a reduction in the number of polar and/or charged groups on the surface, and wherein at least 40% of the pore volume of the activated carbon-based sorbent results from pores having diameters in the range of from about 15Å to about 50Å; and

c) recovering at least a portion of the removed quantity from the activated carbon-based sorbent forming a recovered quantity; wherein the recovered quantity is combined with the bio-oil stream.

**BRIEF DESCRIPTION OF THE DRAWINGS**

The features and advantages of embodiments of the invention will be illustrated with reference to the following drawings. The drawings are not to scale and certain features are shown exaggerated in scale or in schematic form in the interest of clarity and conciseness.

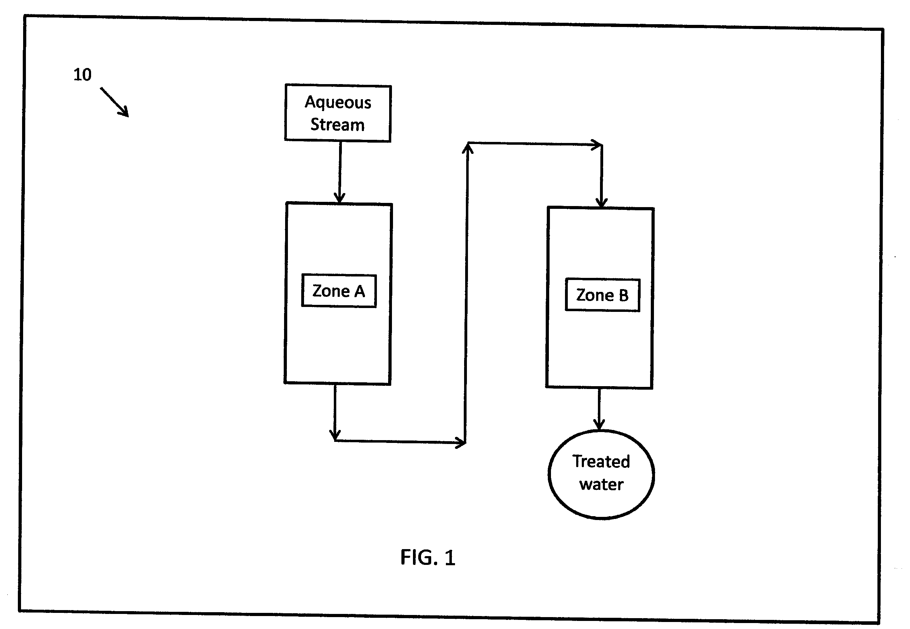

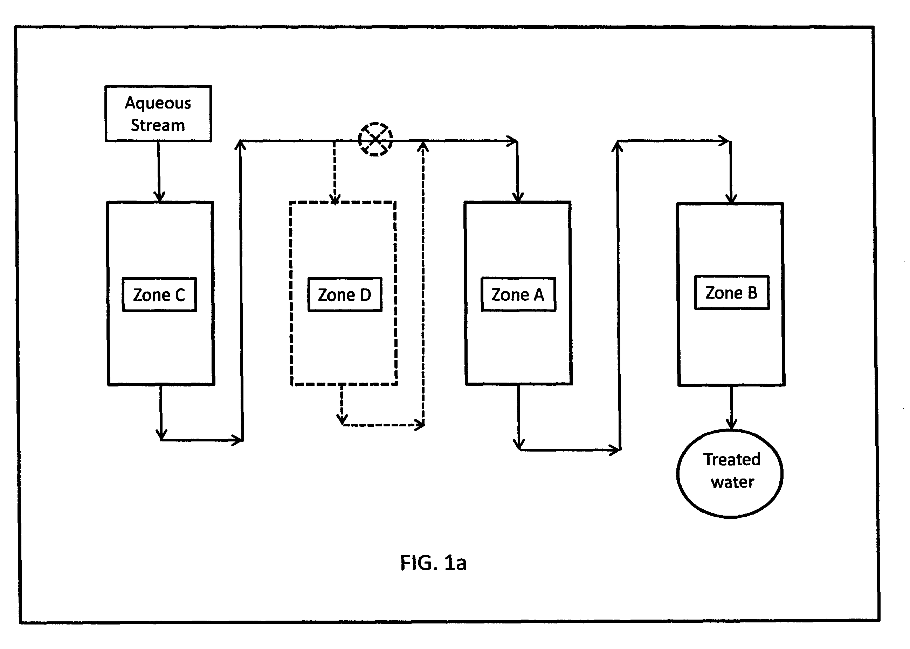

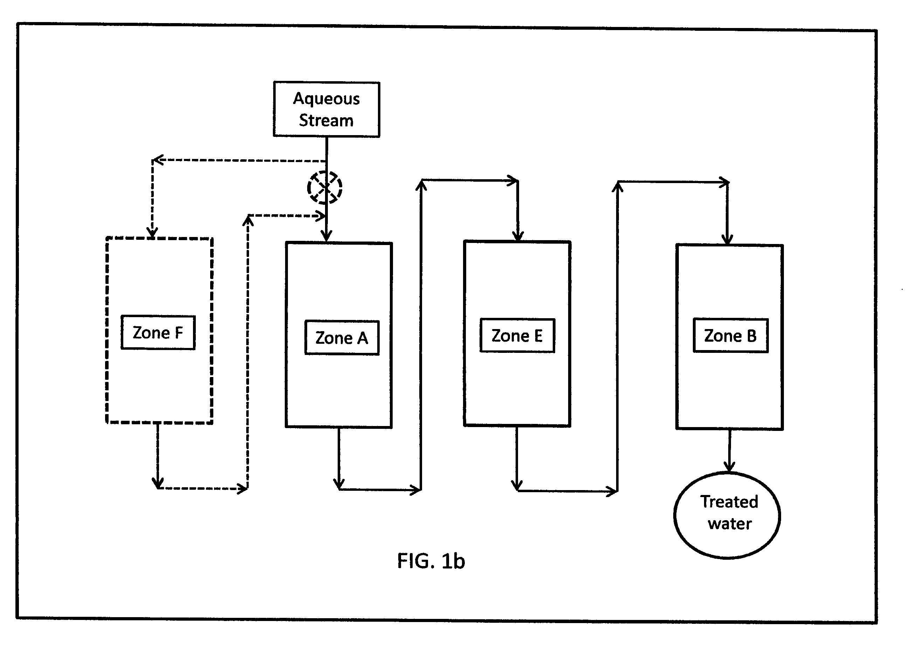

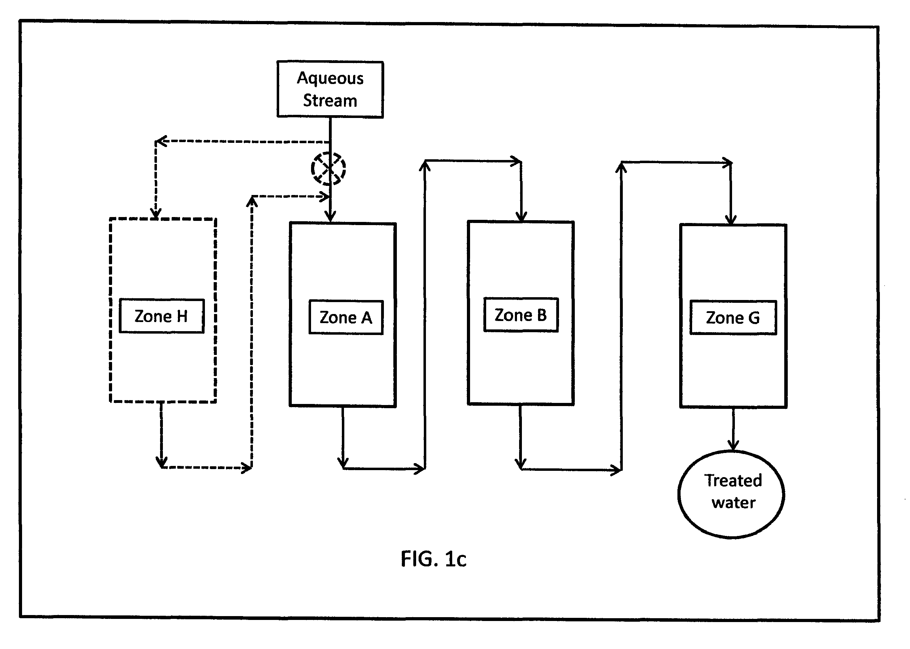

FIG. 1 is a schematic view of a two-stage sorption method/system for carrying out a specific embodiment of the invention.

FIG. 1 a is a schematic view of a two-stage sorption method/system including up to two additional optional stages for carrying out specific embodiments of the invention.

FIG. 1 b is a schematic view of a two-stage sorption method/system including up to two additional optional stages for carrying out specific embodiments of the invention.

FIG. 1 c is a schematic view of a two-stage sorption method/system including up to two additional optional stages for carrying out specific embodiments of the invention.

FIG. 2 is a schematic view of a sorption method/system including a bio-oil/water separator for carrying out specific embodiments of the invention.

FIG. 3 is a schematic view of a method/system for recovering sorbed quantities from a removal zone in accordance with an embodiment of the invention.

FIG. 4 is a schematic view of a method/system for recovering sorbed quantities from a removal zone in accordance with an embodiment of the invention.

FIG. 5 is a schematic view of a method/system for recovering sorbed quantities from a removal zone in accordance with an embodiment of the invention.

FIG. 6 is a graph depicting thermograms for various granulated activated carbon samples.

**DETAILED DESCRIPTION OF THE INVENTION**

Of the various processes for converting biomass, pyrolysis processes, in particular flash pyrolysis processes, are generally recognized as offering the most promising routes to the conversion of solid biomass materials to liquid products, generally referred to as bio-oil or bio-crude. In addition to liquid reaction products, these processes produce gaseous reaction products and solid reaction products. Gaseous reaction products comprise carbon dioxide, carbon monoxide, and relatively minor amounts of hydrogen, methane, and ethylene. The solid reaction products comprise coke and char.

In order to maximize the liquid yield, while minimizing the solid and non-condensable gaseous reaction products, the pyrolysis process should provide a relatively fast heating rate of the biomass feedstock. Lately, the focus has been on ablative reactors, cyclone reactors, and fluidized reactors to provide the fast heating rates. Fluidized reactors include both fluidized stationary bed reactors and transport reactors.

Transport reactors provide heat to the reactor feed by injecting hot particulate heat carrier material into the reaction zone. This technique provides rapid heating of the feedstock. The fluidization of the feedstock ensures an even heat distribution within the mixing zone of the reactor.

The biomass to be pyrolyzed is generally ground to a small particle size in order to optimize pyrolysis. The biomass may be ground in a grinder or a mill until the desired particle size is achieved.

Regardless of the process used to produce bio-oil from the conversion of biomass, an aqueous stream is produced as a part of the reaction products. In the case of non-catalytic pyrolysis, this aqueous stream is usually emulsified with the organic portion (bio-oil) of the reaction products. In such case, the aqueous stream is only separable from the organic portion of the reaction products upon breaking the emulsion by some further treatment, such as hydrotreatment or deoxygenation, of the organic components of the reaction products. In the case of catalytic pyrolysis of biomass, the aqueous and bio-oil streams form into two separate phases which are separable by methods including, but not limited to, gravity separation and centrifugation. In such case, the bio-oil phase is often more dense than the aqueous phase, causing the aqueous phase to rest on top of the bio-oil phase. In order to flip the layers (resulting in lower solids content for the bio-oil), the density of either the aqueous phase or bio-oil phase can be adjusted. Methods to perform such flipping of layers is disclosed in US Patent Application No. 13/212,861 filed on August 18, 2011, which has been incorporated herein by reference in its entirety.

The aqueous stream, however obtained from the conversion of biomass, can contain up to 10 or up to 15 wt% of a water-soluble complex mixture of organic compounds. This amount of organic compounds can account for up to 30 wt% of the total organics yield from the biomass. The water-soluble complex mixture of organic compounds contained in the aqueous phase include phenols, catechols, aromatics, aldehydes, ketones, carboxylic acids, furans, indenols, and naphthols. Their relative proportions increase with increasing polarity of the compound.

An embodiment of the invention will be described with reference to FIG. 1. A method 10 for recovering at least a portion of the water-soluble complex mixture of organic compounds from the aqueous stream comprises, consists of, or consists essentially of:

a) passing the aqueous stream comprising the water-soluble complex mixture of organic compounds to a removal zone A for contact with a sorbent A comprising a polymeric microreticular sorbent resin contained therein for removal of at least a portion of the water-soluble complex mixture of organic compounds from the aqueous stream forming a removed quantity A comprising water-soluble organic compounds; and

b) passing the aqueous stream from the removal zone A to a removal zone B for contact with a sorbent B contained therein for removal of at least a portion of the water-soluble complex mixture of organic compounds from the aqueous stream forming a removed quantity B comprising water-soluble organic compounds and a treated water stream.

At least a portion of the removed quantity A can be recovered from the removal zone A forming a recovered quantity A and at least a portion of the removed quantity B can be recovered from the removal zone B forming a recovered quantity B.

Sorbent B can be selected from the group consisting of polymeric microreticular sorbent resins, zeolite-based adsorbents, clay-based adsorbents, activated carbon-based sorbents, and mixtures thereof. The polymeric microreticular sorbent resins can be selected from the group consisting of Amberlite, Osorb, Amberlyst, Super adsorbent, and mixtures thereof; the zeolite-based adsorbents can be selected from the group consisting of X-Faujasite, Y-Faujasite, ZSM-5, zeolite-A, and mixtures thereof; the clay-based adsorbents can be selected from the group consisting of kaolin, bentonite, chlorite, perovskite, smectite, organoclays and mixtures thereof; and the activated carbon-based sorbents can be selected from the group consisting of microporous activated carbon, mesoporous activated carbon, carbon molecular sieves, carbon microbeads, carbon powder, granular activated carbon, and mixtures thereof.

With further reference to FIGs 1 and 1a, the aqueous stream can optionally be passed to a removal zone C prior to being passed to removal zone A. In removal zone C, at least a portion of the water-soluble complex mixture of organic compounds can be removed from the aqueous stream forming a removed quantity C comprising water-soluble organic compounds. At least a portion of the removed quantity C can be removed from the removal zone C forming recovered quantity C.

Removal zone C can comprise a sorbent C selected from the group consisting of zeolite-based adsorbents (as described above), clay-based adsorbents, (as described above), and mixtures thereof, which sorbs at least a portion of the water-soluble complex mixture of organic compounds from the aqueous stream through contact with sorbent C forming the removed quantity C.

With further reference to FIGs 1 and 1a, the aqueous stream can optionally be passed from removal zone C to a removal zone D comprising a super absorbent polymer for removal of at least a portion of the water from the aqueous stream through contact with the super absorbent polymer and yielding a stream of recovered quantity D comprising concentrated water-soluble organic compounds. The stream of recovered quantity D can then be passed to removal zone A. Also, the super absorbent polymer in removal zone D can be regenerated by taking removal zone D offline and heating at a temperature between about 50°C and about 90°C under an inert gas flow having a GHSV of at least about $0.5h^{-1}$, thereby removing the water.

Removal zone C can comprise an extraction zone wherein the aqueous stream is contacted with a solvent selected from the group consisting of i) renewable gasoline, ii) toluene, iii) xylene, iv) oxygenated solvents selected from the group consisting of methanol, ethanol, isopropanol, acetone, methylbutyl ketone, tetrahydrofuran, ethyl acetate, and v) mixtures thereof for extractive removal of at least a portion of the water-soluble complex mixture of organic compounds from the aqueous stream forming the removed quantity C.

With reference to FIGs 1 and 1 b, the aqueous stream can optionally be passed from removal zone A to a removal zone E comprising a super absorbent polymer prior to being passed to removal zone B. At least a portion of the water from the aqueous stream can be removed in removal zone E through contact with the super absorbent polymer and yielding a stream of recovered quantity E comprising concentrated water-soluble organic compounds. The stream of recovered quantity E can then be passed from removal zone E to removal zone B. Also, the super absorbent polymer in removal zone E can be regenerated by taking removal zone E offline and heating at a temperature between about 50°C and about 90°C under an inert gas flow having a GHSV of at least about $0.5h^{-1}$, thereby removing the water.

With further reference to FIGs 1 and 1b, the aqueous stream can optionally be passed to a removal zone F comprising a zeolite-based adsorbent prior to being passed to removal zone A. At least a portion of the water-soluble complex mixture of organic compounds are sorbed from the aqueous stream through contact with the zeolite-based adsorbent forming a removed quantity F comprising water-soluble organic compounds. At least a portion of the removed quantity F can be removed from the removal zone F forming recovered quantity F.

With reference to FIGs 1 and 1c, the aqueous stream can optionally be passed from removal zone B to a removal zone G comprising a super absorbent polymer. At least a portion of the water from the aqueous stream can be removed in removal zone G through contact with the super absorbent polymer and yielding a stream of recovered quantity G comprising concentrated water-soluble organic compounds. Also, the super absorbent polymer in removal zone G can be regenerated by taking removal zone G offline and heating at a temperature between about 50°C and about 90°C under an inert gas flow having a GHSV of at least about $0.5h^{-1}$, thereby removing the water.

With further reference to FIGs 1 and 1c, the aqueous stream can optionally be passed to a removal zone H comprising a sorbent H prior to being passed to removal zone A. Sorbent H can be selected from the group consisting of zeolite-based adsorbents, clay-based adsorbents, and mixtures thereof. At least a portion of the water-soluble organic compounds from the aqueous stream can be sorbed in removal zone H through contact with the sorbent H forming a removed quantity H comprising water-soluble organic compounds. At least a portion of the removed quantity H can be removed from the removal zone H forming recovered quantity H.

Optionally, a bio-oil/water stream comprising a water-soluble complex mixture of organic compounds, water-insoluble organic compounds, and water is separated into a bio-oil stream comprising water-insoluble organic compounds and into the aqueous stream. The recovered quantities (recovered from the aqueous stream) described above can be combined with the bio-oil stream.

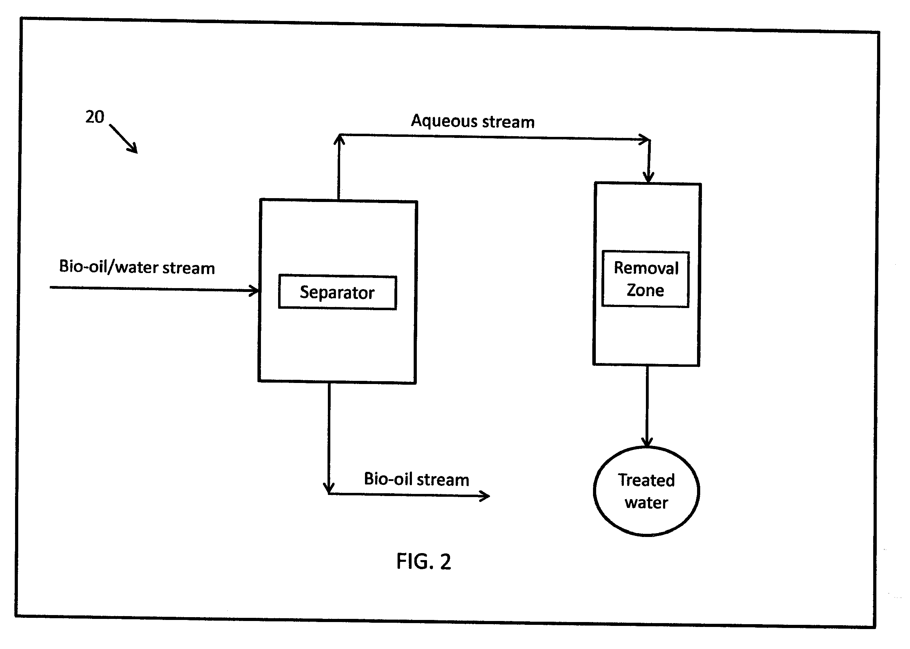

An embodiment of the invention will be described with reference to FIG. 2. A method 20 for recovering a water-soluble complex mixture of organic compounds from an aqueous stream comprises, consists of, or consists essentially of:

a) separating a bio-oil/water stream comprising a water-soluble complex mixture of organic compounds, water-insoluble organic compounds, and water in a separator into a bio-oil stream comprising water-insoluble organic compounds and into the aqueous stream comprising a water-soluble complex mixture of organic compounds;

b) contacting the aqueous stream with an activated carbon-based sorbent in a removal zone for removal of at least a portion of the water-soluble complex mixture of organic compounds from the aqueous stream forming a removed quantity comprising water-soluble organic compounds and a treated water stream, wherein the activated carbon-based sorbent has been surface treated in a manner resulting in a reduction in the number of polar and/or charged groups on the surface, and wherein at least about 40% of the pore volume of the activated carbon-based sorbent results from pores having diameters in the range of from about 15Å to about 50Å.

At least a portion of the removed quantity can be recovered from the activated carbon-based sorbent forming a recovered quantity, which can be combined with the bio-oil stream.

The aqueous stream, and intermediate streams described above, can be charged to the removal zones in either an upflow or a downflow mode.

With reference to FIGs 1, 1 a, 1b, 1c, and 2, the method of recovering the removed quantities from the sorbent materials can be any of the methods well known in the art, such as thermal desorption, thermal expansion (under vacuum) or chemical displacement.

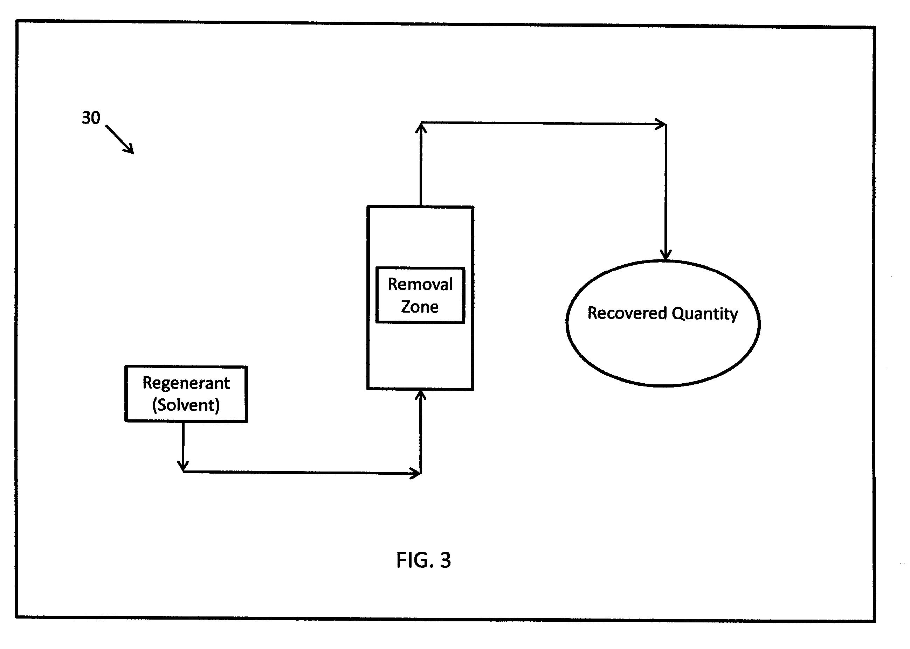

In accordance with an embodiment of the invention, a method 30 for recovering removed quantities from a removal zone is described below with reference to FIG. 3. When the sorbent in a removal zone 300 is selected from the group consisting of at least one of the polymeric microreticular sorbent resins, at least one of the activated carbon-based sorbents, and mixtures thereof, the recovery of the removed quantities as variously described above is carried out by chemical displacement at temperatures in the range of about 20°C to about 200°C or about 30°C to about 150°C or about 40°C to about 100°C using a regenerant (solvent) selected from the group consisting of i) renewable gasoline, ii) toluene, iii) xylene, iv) an oxygenated solvent selected from the group consisting of methanol, ethanol, isopropanol, acetone, methylbutyl ketone, methylisobutyl ketone, tetrahydrofuran, ethyl acetate, and v) mixtures thereof. The regenerant is charged to the removal zone displacing the removed quantity from the sorbent, and the removed quantity is recovered forming a recovered quantity. The regenerant can then be displaced from the sorbent by any suitable manner in order to prepare the removal zone for further removal of water-soluble organic compounds from the aqueous stream, once put back on-line.

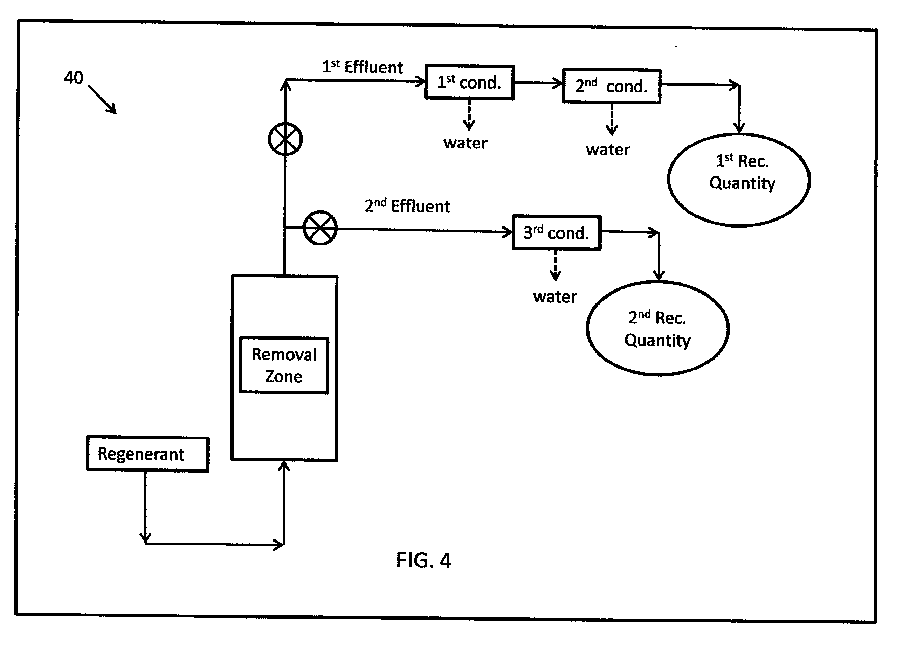

In accordance with an embodiment of the invention, a method 40 for recovering removed quantities from a removal zone is described below with reference to FIG. 4. When the sorbent in the removal zone is selected from the group consisting of at least one of the zeolite-based adsorbents, at least one of the clay-based adsorbents, at least one of the activated carbon-based sorbents, and mixtures thereof, the recovery of the removed quantities as variously described above is carried out by thermal desorption in accordance with the following.

i) The sorbent in the removal zone is heated to a temperature in the range of from about 20°C to about 200°C or about 50°C to about 150°C or about 60°C to about 130°C, by introduction of a heated regenerant, which can be an inert gas, to the removal zone at pressures slightly above atmospheric pressure. A first effluent is removed from the removal zone and is partially condensed in a first condenser at a temperature in the range of from about 20°C to about 50°C, for a period of time between about 0.2 to about 6 hours or about 0.5 to about 4 hours, followed by passing the first effluent to a second condenser wherein the first effluent is further partially condensed at a temperature in the range of from about -150°C to about -30°C or about -100°C to about -40°C for a period of time between about 0.2 to about 6 hours or about 0.5 to about 4 hours, forming a first recovered quantity.

ii) Thereafter, the sorbent is heated to a temperature in the range of from about 130°C to about 500°C or about 150°C to about 400°C or about 200°C to about 350°C, under at least a partial vacuum and for a period of time between about 0.2 to about 6 hours or about 0.5 to about 4 hours. The vacuum can be up to about 0.01 or up to about 0.1 or up to about 1 torr. A second effluent is removed from the removal zone and is passed to a third condenser wherein the second effluent is partially condensed at a temperature in the range of from about -150°C to about -30°C or about -100°C to about -40°C, forming a second recovered quantity. Condensed water can be drawn off from each of the first, second, and third condensers.

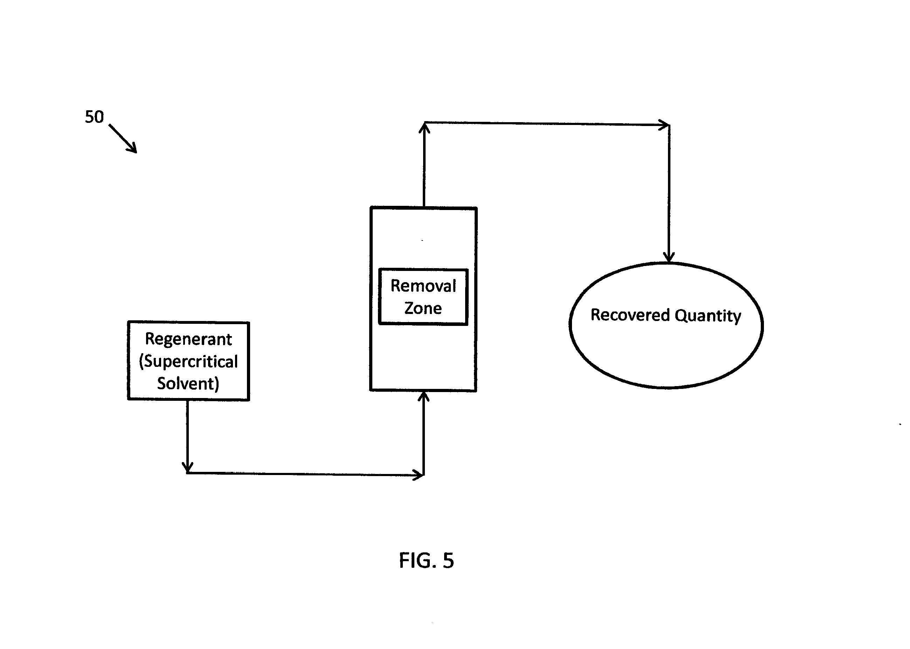

In accordance with an embodiment of the invention, a method 50 for recovering removed quantities from a removal zone is described below with reference to FIG. 5. When the sorbent in the removal zone is selected from the group consisting of at least one of the activated carbon-based sorbents, the recovery of the removed quantity is carried out by chemical displacement using a regenerant which is a supercritical solvent selected from the group consisting of supercritical $CO_2$, supercritical propane, supercritical butane, supercritical toluene, supercritical xylene, and mixtures thereof. The regenerant is charged to the removal zone displacing the removed quantity from the sorbent, and the removed quantity is recovered forming a recovered quantity. The regenerant can then be displaced from the sorbent by any suitable manner in order to prepare the removal zone for further removal of water-soluble organic compounds from the aqueous stream, once put back on-line.

The regenerant streams described above can be charged to the removal zones for recovery of the recovered quantities in either an upflow or a downflow mode.

**EXAMPLES**

Example 1:

Southern yellow pine wood particles were thermocatalytically converted to a reaction product, which was then separated into a bio-oil stream and into an aqueous stream containing about 10 wt% water-soluble organic compounds. A volume of 25 ml of the aqueous stream was then contacted with about 2.5 g of fresh granulated activated carbon (fresh GAC) resulting in a spent GAC containing the water-soluble organic compounds. A 5.06 g quantity of the spent GAC was filtered off from the aqueous phase and subjected to vacuum desorption in a vacuum oven under a vacuum of about 0.1 torr and at a temperature of 120°C, to form vacuum regenerated GACs. A similar experiment was carried out in parallel and the 5.01 g of filtered off spent GAC was subjected to vacuum desorption in such vacuum oven under a vacuum of about 0.1 torr and at a temperature of 50°C. The results of such vacuum desorption are shown in Table 1 below. For the 120°C run, the change in weight loss % from 60 minutes to 120 minutes of only about 0.5% (100*(44.83-44.59)/44.59) shows that the recoverable material from the spent GAC was substantially completely recovered after two hours treatment. Table 1 also shows that at 30 minutes for the 120°C run 98% of the recoverable material was desorbed. For the 50°C run, Table 1 shows that after 180 minutes 93% of the recoverable material was desorbed. Thus, given enough time for desorption, this data shows that vacuum desorption at temperatures as low as 50°C is effective in removing recoverable water-soluble organic compounds from a spent GAC.

**Table 1**

| Conditions | 120°C Vacuum Oven | | 50°C Vacuum Oven | |
| --- | --- | --- | --- | --- |
| Total Time (min) | GAC Mass (g) | Weight loss, % | GAC Mass (g) | Weight loss, % |
| 0 | 5.06 | 0 | 5.01 | 0 |
| 30 | 2.84 | 43.87 | 4.04 | 19.38 |
| 60 | 2.80 | 44.59 | 3.29 | 34.41 |
| 120 | 2.79 | 44.83 | --- | |
| 180 | --- | | 2.93 | 41.52 |

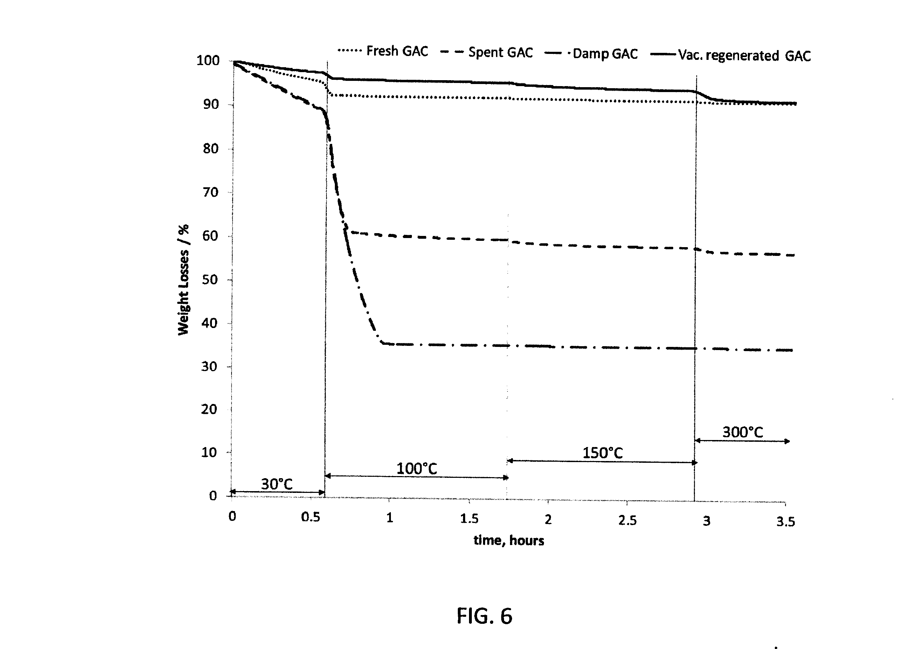

Thermogravimetric analysis (TGA) thermograms were obtained for samples of the spent GAC, the vacuum regenerated GAC from the 120°C run, fresh GAC, and a fresh GAC made damp with DI water (damp GAC). The thermograms are shown in FIG. 6. As can be seen in FIG. 6, the weight loss for the spent GAC confirm the efficiency of thermal regeneration shown in Table 1, and that of the vacuum regenerated GAC was very low and similar to that of the fresh GAC, indicating vacuum regeneration is very effective at removing these water-soluble organic compounds from spent GAC.

Example 2:

A model aqueous solution was prepared containing 0.5 wt% phenol and about 1 wt% catechol. About 50 g quantity of this solution was charged at a flow rate of 1ml/min to a vessel containing about 10 g of a mixture of sorbent resins referred to generally as Amberlite XAD (75% of the resin with product designation Amberlite XAD 761 and 25% of the resin with product designation Amberlite XAD 1600) which are manufactured by the Rohm and Haas company. The resulting spent sorbent resin mixture contained 0.16 g of phenol and 0.45 g of catechol. The spent sorbent resin mixture was subjected to desorption by extraction with about 8 ml ethanol. For both phenol and catechol, Table 2 below shows the wt% removal from the model solution, the wt% desorption from the sorbent resin mixture, and the total overall recovery. The data in Table 2 show that the Amberlite XAD resins are effective in removing phenol and catechol from an aqueous stream, and that such absorbed components can be effectively removed by chemical displacement with a suitable solvent from such resins.

**Table 2**

| Phenol | | | Catechol | | |
|---|---|---|---|---|---|
| Wt% Removal | Wt% Desorption | Wt% Total Recovery | Wt% Removal | Wt% Desorption | Wt% Total Recovery |
| 94.8 | 48.5 | 46.0 | 71.4 | 60.6 | 43.3 |

For the following Examples 3, 4, 5 and 6, aqueous streams containing from about 7 to about 14 wt% water-soluble organic compounds were separated from reaction products resulting from the thermocatalytic conversion of southern yellow pine wood particles. The aqueous streams were then contacted with various sorbents for removal of water-soluble organic compounds, or in the case of Super Absorbent Polymer (SAP) the removal of water resulting in a concentrating effect of the water-soluble organic compounds.

Example 3:

Organics were removed from the aqueous stream following the scheme:

Feed → NaX zeolite → Osorb resin →Amberlyst resin

Table 3 below shows the selectivities for these sorbents in the removal of specific water-soluble organic compounds. The removal %'s for each sorbent is based on the amounts of the subject organics contained in the feed to that in the treated water. The data show that NaX zeolite is very effective in removing heavier compounds, Osorb resin is effective in removing phenolics, and that Amberlyst 21 resin is effective in removing acidic compounds.

**Table 3**

| | Removal, wt% | | |
|---|---|---|---|
| Compound Families | NaX Zeolite | Osorb resin | Amberlyst resin |
| Ketones and Aldehydes | 0 | 49 | 0 |
| Carboxylic acids | 56 | 38 | 86 |
| Phenolics | 71 | 84 | 100 |
| Unidentified heavy compounds | 88 | 100 | N/A |

Example 4:

Tables 4a, 4b, and 4c below show the total removal of water-soluble organic compounds from such aqueous streams described above resulting from the serial contact of the aqueous streams with different sequences and combinations of sorbents. As can be seen from Table 4a, the arrangement used in Run C demonstrated a very selective separation, and in consideration of the selectivities of the sorbents used (shown in Table 3), leaving most of the heavy compounds on the zeolite sorbent, most of the hydroxylic compounds on the Amberlyst sorbent and produced a concentrated ketones and aldehydes stream. The removal wt% for ketones and aldehydes in Run C is negative due to the manner in which the removal %'s are calculated, that is:

$$[(\text{amount in the feed}) - (\text{amount in the effluent})]/(\text{amount in the feed})$$

In the case of Run C, the SAP removes substantial amounts of water, thus concentrating the water-soluble organics not removed upstream by the zeolite. The hydroxylic compounds are then mostly removed by the following Amberlyst resin, leaving a concentrated stream of ketones and aldehydes having a concentration in the final effluent which is higher than the concentration in the feed due to the removal of water by the SAP. Similarly, comparing D and E data shown in Table 4b confirmed the advantageous effectiveness of using SAP in that last stage.

**Table 4a**

| | Removal, wt% | | |
|---|---|---|---|
| RUN | A | B | C |
| Compound Families | Osorb resin/ Amberlyst resin | NaX Zeolite/ Osorb resin/ Amberlyst resin | NaX Zeolite/ SAP/ Amberlyst resin |
| Ketones and Aldehydes | 80 | 16 | -38 |
| Carboxylic acids | 98 | 96 | 99 |
| Phenolics | 98 | 100 | 100 |
| Unidentified heavy compounds | 92 | 100 | 100 |

**Table 4b**

| | Removal, wt% | | |
|---|---|---|---|
| RUN | D | E | F |
| Compound Families | Osorb resin/ SAP/ Amberlyst resin | Osorb resin/ Amberlyst resin/ SAP | NaX-Zeolite/Osorb resin/SAP/Amberlyst resin |
| Ketones and Aldehydes | 58 | 98 | 26 |
| Carboxylic acids | 97 | 98 | 100 |
| Phenolics | 100 | 92 | 100 |
| Unidentified heavy compounds | 64 | 100 | 100 |

Example 5:

Table 5 below shows the total removal and total recovery percentages of water-soluble organic compounds from an aqueous stream as described above resulting from the contact of the aqueous stream, in a column containing a commercial granulated activated carbon (GAC) manufactured by Norit Inc. and having product designation GAC300. The adsorber column was loaded in the downflow mode. Organic compounds were recovered from the GAC through thermal desorption carried out by:

1) establishing a nitrogen flow through the column and heating the column to ramped temperatures of about 90°C, then about 100°C, and then about 150°C, condensing and recovering organics from the effluent from the column in a first flask (condenser) at room temperature (about 21 °C) followed by further condensation and recovery of organics from the effluent in a second condenser at a temperature of about -78°C, and disconnecting the first and second condensers upon no further organics collection; and

2) heating the column to ramped temperatures of about 150°C up to about 190°C under nitrogen flow, and condensing and recovering organics from the effluent from the column in a third condenser connected to a vacuum pump and at a temperature of about -78°C.

The data in Table 5 shows that microporous GAC is effective in removing substantially all of the organics present and is selective for the recovery of the light organic compounds from an aqueous stream. The data also shows that such absorbed light components can be effectively removed from the GAC by thermal desorption.

**Table 5**

| Compound | Removal, wt% | Recovery, wt% | |
|---|---|---|---|
| | | | Grouped Totals |
| **Lights** | | | |
| Methyl Vinyl Ketone | 81 | 59 | |
| 2-Butanone | 99 | 95 | |
| Acetic Acid | 70 | 107 | 62 (Total for lights) |
| Benzene | 100 | 29 | |
| 1-Hydroxy-2-pentanone | 78 | 17 | |
| Propionic Acid | 97 | 64 | |
| **Heavies** | | | |
| Toluene | 79 | 21 | |
| 2-Cyclopentan-1-one | 93 | 16 | |
| Phenol | 100 | 1 | |
| o-Cresol | 100 | 0 | 5 (Total for heavies) |
| (p+m)-Cresol | 100 | 0 | |
| Catechol | 100 | 0 | |
| 1,4-Benzenediol | 100 | 0 | |
| 4-Methyl-1,2-benzediol | 100 | 0 | |
| **Total for all organics** | 93 | 29 | 33 |

Example 6:

Table 6 below shows the total removal and total recovery percentages of water-soluble organic compounds from an aqueous stream as described above resulting from the contact of the aqueous stream, in a column, with: 1) a mixture of sorbent resins referred to generally as Amberlite XAD (75% of the resin with product designation Amberlite XAD 761 and 25% of the resin with product designation Amberlite XAD 1600) which are manufactured by the Rohm and Haas company, followed by contact with: 2) a commercial granulated activated carbon (GAC) manufactured by Norit Inc. and having product designation GAC300. The bottom 25 vol% of the column was packed with the GAC and the top 75 vol% of the column was packed with the Amberlite XAD resin mixture. The adsorber column was loaded in the downflow mode. The spent adsorbents bed was subjected to desorption for recovery of organic compounds by extraction with ethanol in the upflow mode. Such recovery method was expected to be effective with regard to organics recovery from the Amberlite XAD sorbent, but have only minimal effectiveness in organics recovery from the GAC.

The data in Table 6 shows that the use of a two-stage system including Amberlite XAD resins and GAC is effective in removing and recovering organic compounds, including phenol and catechol, from an aqueous stream, and that such absorbed heavy components can be effectively removed from the Amberlite XAD by chemical displacement with a suitable solvent. The light compounds contained in the GAC can be recovered from the GAC by thermal desorption, as described in Examples 1 and 5.

**Table** 6

| Compound | Removal, wt% | Recovery, wt% | |
|---|---|---|---|
| | | | Grouped Totals |
| **Lights** | | | |
| Formaldehyde | 78.7 | 10.7 | |
| Acetaldehyde | 95.3 | 22.1 | |
| Methyl Vinyl Ketone | 90.9 | 50.4 | |
| 2-Butanone | 92.7 | 64.0 | 36.2 (Total for lights) |
| Acetic Acid | 92.7 | 15.0 | |
| Benzene | 98.4 | 73.6 | |
| 1-Hydroxy-2-pentanone | 92.0 | 26.9 | |
| Propionic Acid | 99.6 | 26.9 | |
| **Heavies** | | | |
| Toluene | 98.1 | 39.7 | |
| 2-Cyclopentan-1-one | 99.7 | 30.0 | |
| Phenol | 100 | 39.2 | |
| o-Cresol | 100 | 60.7 | 47.3 (Total for heavies) |
| (p+m)-Cresol | 100 | 55.5 | |
| Catechol | 100 | 34.6 | |
| 1,4-Benzenediol | 100 | 93.5 | |
| 4-Methyl-1,2-benzediol | 84.9 | 25.2 | |
| **Total for all organics** | 93.6 | 29.6 | |

Further, unless expressly stated to the contrary, "or" refers to an inclusive or and not to an exclusive or. For example, a condition A or B is satisfied by anyone of the following: A is true (or present) and B is false (or not present), A is false ( or not present) and B is true ( or present), and both A and B are true (or present).

Further, unless expressly stated otherwise, the term "about" as used herein is intended to include and take into account variations due to manufacturing tolerances and/or variabilities in process control.

Changes may be made in the construction and the operation of the various components, elements and assemblies described herein, and changes may be made in the steps or sequence of steps of the methods described herein without departing from the spirit and the scope of the invention as defined in the following claims.

**Claims**

1. A method for recovering a water-soluble complex mixture of organic compounds from an aqueous stream comprising:

    a) passing said aqueous stream comprising a water-soluble complex mixture of organic compounds to a removal zone A for contact with a sorbent A comprising a polymeric microreticular sorbent resin for removal of at least a portion of the water-soluble complex mixture of organic compounds from said aqueous stream forming a removed quantity A comprising water-soluble organic compounds;
    b) passing said aqueous stream from said removal zone A to a removal zone B for contact with a sorbent B for removal of at least a portion of the water-soluble complex mixture of organic compounds from said aqueous stream forming a removed quantity B comprising water-soluble organic compounds; and
    c) recovering at least a portion of said removed quantity A from said removal zone A forming recovered quantity A and recovering at least a portion of said removed quantity B from said removal zone B forming recovered quantity B.

2. The method of claim 1 wherein said polymeric microreticular sorbent resin of sorbent A is selected from the group consisting of Amberlite, Osorb, Amberlyst, Super adsorbent, and mixtures thereof.

3. The method of claim 1 wherein a bio-oil/water stream comprising a water-soluble complex mixture of organic compounds, water-insoluble organic compounds, and water is separated into a bio-oil stream comprising water-insoluble organic compounds and into said aqueous stream; and wherein said recovered quantities A and B recovered in step c) are combined with said bio-oil stream.

4. The method of claim 1 wherein said sorbent B is selected from the group consisting of polymeric microreticular sorbent resins, zeolite-based adsorbents, clay-based adsorbents, activated carbon-based sorbents, and mixtures thereof.

5. The method of claim 4 wherein said polymeric microreticular sorbent resins are selected from the group consisting of Amberlite, Osorb, Amberlyst, Super adsorbent, and mixtures thereof; and/or
wherein said zeolite-based adsorbents are selected from the group consisting of X-Faujasite, Y-Faujasite, ZSM-5, zeolite-A, and mixtures thereof; and/or wherein said clay-based adsorbents are selected from the group consisting of kaolin, bentonite, chlorite, perovskite, smectite, organoclays and mixtures thereof; and/or
wherein said activated carbon-based sorbents are selected from the group consisting of microporous activated carbon, mesoporous activated carbon, carbon molecular sieves, carbon microbeads, carbon powder, granular activated carbon, and mixtures thereof.

6. The method of claim 4 wherein the recovering of removed quantities in step c), when from a sorbent selected from the group consisting of at least one of said polymeric microreticular sorbent resins, is carried out by chemical displacement at temperatures in the range of about 20°C to about 200°C using a solvent selected from the group consisting of i) renewable gasoline, ii) toluene, iii) xylene, iv) an oxygenated solvent selected from the group consisting of methanol, ethanol, isopropanol, acetone, methylbutyl ketone, tetrahydrofuran, ethyl acetate, and v) mixtures thereof; and/or
wherein the recovering of removed quantities in step c), when from a sorbent selected from the group consisting of at least one of said zeolite-based adsorbents, at least one of said clay-based adsorbents, at least one of said activated carbon-based sorbents, and mixtures thereof, is carried out by thermal desorption by:

i) heating said sorbent to a temperature in the range of from about 20°C to about 150°C, under an inert gas stream at up to atmospheric pressure, and partially condensing the resulting first effluent at a temperature in the range of from about 20°C to about 40°C for a period of time between about 0.5 to about 4 hours, followed by partial condensation at a temperature in the range of from about -100°C to about -50°C for a period of time between about 0.5 to about 4 hours, forming a first recovered quantity; and
ii) thereafter heating said sorbent to a temperature in the range of from about 150°C to about 500°C under at least a partial vacuum and for a period of time between about 0.5 to about 4 hours, and partially condensing the resulting second effluent at a temperature in the range of from about -100°C to about - 50°C, forming a second recovered quantity; and/or

wherein the recovering of removed quantities in step c), when from a sorbent selected from the group consisting of at least one of said activated carbon-based sorbents, is carried out by chemical displacement using a supercritical solvent selected from the group consisting of supercritical $CO_2$, supercritical propane, supercritical butane, supercritical toluene, supercritical xylene, and mixtures thereof.

7. The method of claim 4 wherein, prior to step a), said aqueous stream is passed to a removal zone C for removal of at least a portion of the water-soluble complex mixture of organic compounds from said aqueous stream forming a removed quantity C comprising water-soluble organic compounds; and wherein step c) further comprises recovering at least a portion of said removed quantity C from said removal zone C forming recovered quantity C.

8. The method of claim 7 wherein said removal zone C comprises a sorbent C selected from the group consisting of zeolite-based adsorbents, clay-based adsorbents, and mixtures thereof, which sorbs at least a portion of the water-soluble complex mixture of organic compounds from said aqueous stream through contact with said sorbent C forming said removed quantity C.

9. The method of claim 8 wherein, prior to step a), said aqueous stream is passed from removal zone C to a removal zone D comprising a super absorbent polymer for removal of at least a portion of the water from said aqueous stream through contact with said super absorbent polymer and yielding a stream of recovered quantity D comprising concentrated water-soluble organic compounds; and further comprising regenerating said super absorbent polymer by heating at a temperature between about 50°C and about 90°C under an inert gas flow having a GHSV of at least about $0.5h^{-1}$

10. The method of claim 7 wherein said removal zone C comprises an extraction zone wherein said aqueous stream is contacted with a solvent selected from the group consisting of i) renewable gasoline, ii) toluene, iii) xylene, iv) oxygenated solvents selected from the group consisting of methanol, ethanol, isopropanol, acetone, methylbutyl ketone, tetrahydrofuran, ethyl acetate, and v) mixtures thereof for extractive removal of at least a portion of the water-soluble complex mixture of organic compounds from said aqueous stream forming said removed quantity C.

11. The method of claim 4 wherein, following step a) and prior to step b), said aqueous stream is passed to a removal zone E comprising a super absorbent polymer for removal of at least a portion of the water from said aqueous stream through contact with said super absorbent polymer and yielding a stream of recovered quantity E comprising concentrated water-soluble organic compounds; and further comprising regenerating said super absorbent polymer by heating at a temperature between about 50°C and about 90°C under an inert gas flow having a GHSV of at least about $0.5h^{-1}$;
and preferably wherein, prior to step a), said aqueous stream is passed to a removal zone F comprising a zeolite- based adsorbent which sorbs at least a portion of the water-soluble complex mixture of organic compounds from said aqueous stream through contact with said zeolite-based adsorbent forming a removed quantity F comprising water-soluble organic compounds; and wherein step c) further comprises recovering at least a portion of said removed quantity F from said removal zone F forming recovered quantity F.

12. The method of claim 4 wherein, following step b), said aqueous stream is passed to a removal zone G comprising a super absorbent polymer for removal of at least a portion of the water from said aqueous stream through contact with said super absorbent polymer and yielding a stream of recovered quantity G comprising concentrated water- soluble organic compounds; and further comprising regenerating said super absorbent polymer by heating at a temperature between about 50°C and about 90°C under an inert gas flow having a GHSV of at least about $0.5h^{-1}$;
and preferably wherein, prior to step a), said aqueous stream is passed to a removal zone H comprising a sorbent H selected from the group consisting of zeolite-based adsorbents, clay-based adsorbents, and mixtures thereof, which sorbs at least a portion of the water-soluble organic compounds from said aqueous stream through contact with said sorbent H forming a removed quantity H comprising water-soluble organic compounds; and wherein step c) further comprises recovering at least a portion of said removed quantity H from said removal zone H forming recovered quantity H.

13. A method for recovering a water-soluble complex mixture of organic compounds from an aqueous stream comprising:

a) separating a bio-oil/water stream comprising a water-soluble complex mixture of organic compounds, water- insoluble organic compounds, and water into a bio-oil stream comprising water-insoluble organic compounds and into said aqueous stream comprising a water-soluble complex mixture of organic compounds;
b) contacting said aqueous stream with an activated carbon-based sorbent for removal of at least a portion of the water-soluble complex mixture of organic compounds from said aqueous stream forming a removed quantity comprising water-soluble organic compounds, wherein the activated carbon-based sorbent has been surface treated in a manner resulting in a reduction in the number of polar and/or charged groups on the surface, and wherein at least 40% of the pore volume of the activated carbon-based sorbent results from pores having diameters in the range of from about 15Å to about 50Å; and
c) recovering at least a portion of said removed quantity from said activated carbon-based sorbent forming a recovered quantity; wherein said recovered quantity is combined with said bio-oil stream.

14. The method of claim 13 wherein step c) is carried out by:

i) recovering a first recovered quantity of said removed quantity from said activated carbon-based sorbent by heating said activated carbon-based sorbent to a temperature in the range of from about 20°C to about 150°C under an inert gas stream at up to atmospheric pressure, and partially condensing the resulting first effluent at a temperature in the range of from about 20°C to about 40°C for a period of time between about 0.5 to about 4 hours, followed by partial condensation at a temperature in the range of from about -100°C to about -50°C for a period of time between about 0.5 to about 4 hours, forming said first recovered quantity; and
ii) thereafter recovering a second recovered quantity of said removed quantity from said activated carbon-based sorbent by heating said activated carbon-based sorbent to a temperature in the range of from about 150°C to about 450°C under at least a partial vacuum and for a period of time between about 0.5 to about 4 hours, and partially condensing the resulting second effluent at a temperature in the range of from about -100°C to about -50°C, forming said second recovered quantity.

15. The method of claim 13 wherein step c) is carried out by chemical displacement using a supercritical solvent selected from the group consisting of supercritical $CO_2$, supercritical propane, supercritical butane, supercritical toluene, supercritical xylene, and mixtures thereof.

FIG. 1

FIG. 1a

FIG. 1b

FIG. 1c

FIG. 2

FIG. 3

FIG. 4

FIG. 5

FIG. 6

EP 2 765 176 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 13212861 B